# EUROPEAN PATENT APPLICATION

(11) **EP 2 275 092 A1**
(43) Date of publication of application: **19.01.2011**
(21) Application number: 08836609.1
(22) Date of filing: 01.10.2008
(51) Int. Cl.: A61K 9/19, A61K 31/337

(54) **TAXANE PHARMACEUTICAL FORMULATION**

(30) Priority: 03.10.2007 AR P070104374
(71) Applicant: Eriochem, S.A., Parana, Entre Rios (AR); Lorenzón, Esteban, Esperanza - Santa Fe S3080AHJ (AR)
(72) Inventor: LORENZÓN, Esteban, Esperanza - Santa Fe S3080AHJ (AR); NUÑEZ, José Lucio, Colonia Avellaneda PARANÀ-ENTRE RIOS (AR); ITURRASPE, José Bernardo, Santa Fe - Santa Fe S3002FOE (AR); MORONI, Guillermo Norberto, Paraná - Entre Rios E3100GOJ (AR)
(74) Representative: Gislon, Gabriele
(86) International application number: PCT/ES2008/070178
(87) International publication number: WO 2009/043956

(57) **Abstract**

A pharmaceutical formulation of taxane to be administered to mammals, preferably humans, comprising two compositions which are combined prior to their administration, forming a transparent solution free from precipitates and essentially free from foam, wherein said compositions comprise a solid composition of taxane and a solubilizing composition of said solid taxane composition comprising a tensoactive and an antifoam agent. A kit for said formulation of injectable taxane comprising a prefilled syringe. And a pharmaceutical taxane perfusion solution.

## Description

### Field of the invention

The present invention relates to the field of pharmaceutical formulations of poorly water-soluble active principles. In particular, it refers to injectable pharmaceutical formulations of taxanes suitable for parenteral infusion processes in oncology chemotherapy.

### State of the Art

Pharmaceutical formulations of active principles poorly soluble in aqueous media have been profusely studied over the last decades. Innumerable strategies have been developed aimed at the injection of these active principles into mammals so as to improve their pharmacotechnics and diminish their side effects.

Regarding pharmaceutical formulations suitable for preparation of solutions of parenteral infusions for prolonged periods, and especially for treatments of oncological chemotherapy, there are technical problems such as maintaining these active principles in the aqueous solution of the parenteral infusion for at least four hours for protocols of conventional infusion and at least 72 hours for administration with continuous infusion pumps. Another problem described is swelling or gelification *in situ,* which originates at the time of the injection of the concentrated solution of the drug, in the container of the parenteral infusion. This event is described as irreversible, in which the tensoactive becomes a gel and does not dissolve in the parenteral solution.

Compounds of the family of taxanes are of a particular interest for the present invention. They are products extracted from the leaves and bark of a tree known with the common name of yew (Taxus baccata and other species of the genus Taxus), such as paclitaxel, as well as artificially semisynthesized compounds obtained from baccatin III or from 10-deacetylbaccatin III, also extracted from yew, such as docetaxel. Those taxanes that are obtained from biotechnological processes are also of interest for the present invention. The medical uses of taxanes are diverse; their antitumoral effects can be mentioned among others. Among cancers that are treated with docetaxel there can be mentioned: locally advanced or metastatic breast cancer, non-small cell lung carcinoma, hormone refractory prostate cancer, ovarian cancer and stomach cancer.

US patent 4814470 by Colin, Michel et al. from the firm Rhone-Poulenc Sante (Courbevoie, FR) refers to a pharmaceutical composition of taxanes, in which derivatives from 10-deacetylbaccatin III, especially docetaxel, are obtained. This document describes a synthesis that ends in crystallization and a formulation that comprises dissolution of the crystals obtained in a mixture of non-ionic tensoactive and alcohol in equal parts. This formulation becomes a gel when injected into bags for parenteral infusion.

Several patents of the firm Rhone-Poulenc Sante (Courbevoie, FR) claim the current formulation of docetaxel on the market(US 5438072, US5698582, US5714512 and US5750561). This technology solves the problems of precipitation and gelification *in situ* by means of a formulation which offers two solutions: one of taxane in polysorbate 80 and alcohol (which may be present in a very low concentration) and the other one consisting of water and a diluting additive which may be ethanol. These documents describe a preparation procedure which consists of diluting the docetaxel of the first solution, mixing without stirring strongly both solutions to generate a solution of Taxotere®, 10 mg/ml (stable between 2 and 25° C for 4 hours), which solves the problem of gelification *in situ* when diluting this solution in an aqueous dextrose solution 5% or normal saline solution (0.9% sodium chloride) to obtain the infusion solution at a concentration of 0.3 to 0.74 mg/ml (stable for 8 hours, between 2 and 25° C).

Said liquid solution of docetaxel in polysorbate 80 (in the presence of alcohol) has stability problems over time. Its conservation in 09^-° C is preferred. Besides, the presence of polysorbate 80 causes known adverse side effects due to the incorporation into the bloodstream of said tensoactive in high concentrations, which are necessary to maintain the drug in solution. The same happens with the formulation of paclitaxel available on the market, which requires high concentrations of Cremophor. The adverse side effects caused by these tensoactives present in taxane formulations available on the market, particularly docetaxel and paclitaxel, require a pre-treatment with steroids or antihistamines before oncological chemotherapy as described in the following scientific works: "Ten Tije AJ, Verweij J, Loos WJ, Sparreboom A. Pharmacological effects of formulation vehicles: Implications for cancer chemotherapy. Clin Pharmacokinet 2003; 42: 665-685"; "Rowinsky EK, Eisenhauer EA, Chaudhry V et al. Clinical toxicities encountered with paclitaxel (Taxol). Semin Oncol 1993, 20: 1-15"; "Bernstein B. Docetaxel as an alternative to paclitaxel after acute hypersensitivity reactions. Ann Pharmacother 2000; 34: 1332-1335"; "Novel formulations of taxanes: a review. Old wine in a new bottle? K. L. Hennenfent1 & R. Govindan Annals of Oncology 17: 735-749, 2006.

Likewise, the formulation of docetaxel available on the market today requires for its use a procedure which involves several steps and a certain risk for the health care professionals involved in its administration. Said steps, aimed at ensuring the proper administration of this drug involve: Extracting the solvent from an ampoule and introducing it into a vial containing the docetaxel solution; gently stirring to homogenize the solution, allowing a standing time for foam to disappear; extracting and injecting the mixture into the perfusion bag or flask containing dextrose or saline solution; homogenizing and finally inspecting for possible precipitates formation before administering it to patients (should precipitation appear, the solution must be disposed of with the resulting economic negative impact).

The whole process must be carried out aseptically. There are high risks of contamination and the operation requires trained personnel and prolonged periods of time. There are also contamination risks for the personnel dealing with the cytotoxic solution, due to the aerosolization of the drug.

The toxic effects of the tensoactives used in the formulation of docetaxel and paclitaxel, such as polyoxyethylated castor oil (Emulphor™ or Cremophor™) or polysorbate 80 (Tween 80™) are known. Pharmacological and biological effects caused by these tensoactives have been described, such as acute hypersensitivity reactions, peripheral neuropathy, cumulative fluid retention syndrome, etc. A great number of patients cannot be treated due to the side effects of said tensoactive, namely patients with hypersensitivity to tensoactives, patients with impaired renal function, elderly patients, cardiac patients, etc. These tensoactives also affect the availability of the active principles solubilized and administered intravenously.

Therefore, great efforts have been made in order to find formulations to avoid or reduce the use of said tensoactives. Among the strategies described in the state of the art, the development of albumin nanoparticles, polyglutamates, taxane analogs, prodrugs, emulsions, liposomes, etc. can be mentioned. For the purpose of reference, scientific work describing in detail the latest developments and clinical tests is provided: "Novel formulations of taxanes: a review. Old wine in a new bottle?" K. L. Hennenfent1 & R. Govindan2 1St Louis College of Pharmacy, Ortho Biotech Clinical Affairs, LLC, St Louis, MO; 2Alvin J Siteman Cancer Center, Washington University School of Medicine, St Louis, MO, USA accepted 7 November 2005. Other works contributing solutions to this problems are: "Castro CA et al., Pharmacol Biochem Behav. 1995 Apr; 50(4):521-6; Sanzgiri UY et al., Fundam Appl Toxicol. 1997 Mar; 36(1):54-61; ten Tije AJ et al., Clin Pharmacokinet. 2003; 42(7):665-85"; "Constantine JW et al., Experientia. 1979 Mar 15; 35(3):338-9"; "Van Zuylen L et al., Invest New Drugs. 2001 May; 19(2): 125-41"; "Bergh M et al., Contact Dermatitis. 1997 Jul; 37(1):9-18)".

The World Health Organization (WHO) has estimated that the maximum daily dose of polysorbate 80 is 25 mg/kg of body weight (FAO WHO. Tech. Rep. Ser. Wid. Hlth. Org. 1974, No 539). Therefore, a man weighing 75 kg may be administered 1.875 g of polysorbate 80 per day; i.e., for a dosage of 75 mg of Taxotere™ per m2 commonly used for lung or prostate cancer, a man 1.75 m tall and 75 kg has a body surface of 1.90 m2 and will receive 143 mg of docetaxel together with approximately 3.6 g of polysorbate 80 (40 mg of docetaxel per ml of Tween 80™), which represents almost twice the amount the maximum daily dosage of polysorbate 80 recommended by the WHO.

There have been several attempts in the state of the art aimed at solving the problem of toxicity of non-ionic tensoactives, proposing taxane lyophilized formulations. For example, patent WO 99/24073 by Géczy, J (Thissen Laboratories S.A.) proposes lyophilizing docetaxel and paclitaxel starting from a hydroalcoholic solution of these drugs and cyclodextrins. This solution enables to obtain a lyophilizate containing taxane complexed to a cyclodextrin as lyophilized powder (it uses 2-hydroxypropyl-β-cyclodextrin for docetaxel in a mass ratio approximately 1:100 of active principle:cyclodextrin). This lyophilizate can be dissolved in an aqueous solution of up to 1 mg/ml, ready for perfusion. Although it manages to avoid the use of non-ionic tensoactives, this formulation incorporates a complex of taxane and cyclodextrin into the bloodstream, which not only increases stability as regards precipitation of a taxane over saturated aqueous solution, but which can also modify the significant pharmacodynamic properties of taxane. Therefore, a toxicological and clinical study to endorse the use of this new complex of taxane and cyclodextrin is required. Besides, complex elaboration processes are involved.

Other attempts to obtain taxane lyophilizates are presented in the state of the art, such as patent application US 20030099674 by Chen, Andrew, in which obtaining a lyophilized taxane from an oil/water emulsion using lecithin as a tensoactive and sucrose as an anti-adhesion agent is proposed. Bile salts are among the surfactants mentioned in the description. Taxane is dissolved in ethanol and water, and the solvents are then eliminated by lyophilization, generating a taxane liposome when reconstituted with water.

On the other hand, US patent application 20030099675 by Jeong, Seo Young, proposes a liquid formulation having an organic solvent, an emulsifier and a monoglyceride. Besides, it proposes forming an emulsion in water from a liquid formulation and lyophilizing it. The use of active drugs such as paclitaxel is mentioned in this document.

In the latter two applications a taxane lyophilized with a good solubility in water is obtained, but it presents similar problems to those mentioned before as regards the use of taxane emulsions which can modify their pharmacodynamics. Naturally, both emulsions or microemulsions and liposomes, when administered endogenously, generate autoimmune responses and would be attacked by macrophages, which should cause an important part of the dosage to be unavailable for the desired action aside from generally requiring pretreatments with steroids or antihistamine.

Other technological developments such as the one described in US patent application 20030187062 by Zenoni Mauricio, et al. of ACS DOBFAR S.A. propose obtaining micro or nanoparticles by lyophilizing paclitaxel and albumin.

Bibliography describing taxane liposomes is abundant, such as EP patent 1332755, in which a lyophilizate of paclitaxel is obtained using a compound such as lecithin or cholesterol in a solution of isopropanol or ethanol in order to subsequently obtain liposomes which are lyophilized, always from aqueous solutions. One of the drawbacks of these developments is that they modify the pharmacodynamics of taxanes, they have a short useful life and require cold chain for their preservation, apart from generating an immune response and causing the attack of macrophages which should considerably reduce the effect of the drug.

Patents claiming polymer micelles are abundant in the bibliography, such as US 5,543,158 and US 6,322,805. In particular, U.S. Pat. No. 6,322,805 refers to obtaining biodegradable polymeric micelles capable of solubilizing hydrophobic drugs comprising amphiphilic block copolymers, which have a hydrophilic polyalkyl oxide component and a biodegradable hydrophobic polymer selected from the group consisting of polylactic acid, polyglycolic acid, poly(lactic-co-glycolic acid),poly(epsilon-caprolactone),their derivatives and mixtures thereof. It describes how the hydrophobic drug is trapped in the micelle in the absence of a covalent bond. These micelles form a solution in water acting as solubilizing agent. This solution can be lyophilized, preserved and reconstituted with water or isotonic solution. This patent does not solve the problem of increasing the solubility of taxane as such, but it presents a complex process of synthesis of a specific copolymer to generate taxane micelles. In spite of having promising tests regarding characteristics of stability, components are added to the drug, radically modifying not only the bioavailability but also the kinetics of the original taxane. This patent does not describe a method to obtain a sterile solution by means of reconstitution.

U.S. Pat. No. 6,780,324 describes a process in which a solution of a biologically active hydrophobic agent is formed in combination with a dispersing agent and an organic solvent or a mixture which can include water. This mixture can be lyophilized and rehydrated to form a nanodispersion or a micelle solution. Drug is not lost in the process. It can be sterilized by filtration; a transparent solution is obtained by reconstitution but the lyophilizate has other components apart from the drug, which involves risks not only for the chemical stability in time but also for its bioavailability. On the other hand, the proposed procedure requires operations such as sonication, intense stirring and heating to obtain the solution to be lyophilized, that can either destroy micelles or cause taxane degradation.

U.S. Pat. No. 6,610,317 B2 by Julie Straub et al. describes a porous matrix of paclitaxel manufactured by mixing taxane dissolved in organic solvent, specifically ethanol, with polysorbate 80, other tensoactives and excipients such as mannitol to further evaporate the solvent by spray drying. This formulation proposes a soluble solid having components in its formulation which can cause side effects such as those generated by polysorbate 80. Besides, it proposes heating the drug for drying, a step which originates degradation products known in the state of the art. On the other hand, the paclitaxel solution that can be manufactured following the teachings of said patent contains 80% of ethanol, which would make a lyophilization process impossible to be applied to generate the solid formulation proposed.

Patent application US 2004/0247624 A1 by Evan Charles Unger, et al. describes another method to manufacture a drug formulation poorly soluble in water. The manufacture of a solid composition is proposed, by lyophilizing a solution of an organic solvent filtered, the poorly soluble drug and at least a stabilizing agent non covalently bonded to the drug. The only example with paclitaxel (Example 3) describes a complex process of sonication and heating until solubilization is reached, of a mixture of two polymers in high concentration, t-butanol and the drug. Then, lyophilization is proposed to obtain a powder. Finally, it describes a redissolution of said powder with another tensoactive solution which finally cannot dissolve the whole solid since it states there are visible particles. Besides, heating the drug in solution at 60° C causes the degradation of paclitaxel.

US application 2005/0152979 by Marc Besman et al. claims a lyophilized composition of a drug poorly soluble in water containing said drug, a polymer and an agent to improve reconstitution. In spite of claiming paclitaxel and docetaxel as drugs suitable to be used by the invention, the tests declared are performed with a paclitaxel conjugate named CT-2103 which is an ester of paclitaxel and alpha-poly-(L) glutamic acid, which has a much higher solubility in aqueous solutions than paclitaxel itself. Neither paclitaxel nor docetaxel dissolve in aqueous solutions of sodium phosphate with the excipients and tensoactives described in this document.

US patent application 2003/0099674 Al by Andrew Chen describes a composition of taxane capable of being dissolved in water manufactured by the lyophilization of an emulsion of the drug in oil, also containing an anti-adhesion agent. An important degradation of taxanes in oils is observed in this patent, when they are subjected to a temperature of 60° C for a month. This technology has the problems described regarding stability and macrophage reactions in the presence of emulsions injected in the body of mammals.

Other technologies such as those used in taxane conjugates are described in documents such as US patent application 20030147807 by Chun Li et al. which describes a composition of taxane soluble in water, but it refers to conjugates of paclitaxel and docetaxel linked to soluble polymers such as polyglutaminic acid, polyaspartic acid, etc.

Other patents evaluated as the state of the art related to the present invention were the following: US2005/0191323, WO9814174, US6630121, US6607784, WO2005/044225, US 2006/0127420, US2006/0078619, US2004/0091541, US2003/0215496, US2001/0018072, US5922754, WO2005025499

To achieve solubilization of these active pronciples having poor solubility in aqueous media, a great number of methods have been proposed in the previous state of the art, such as intense stirring, heating, sonication, solvent evaporation, dialysis, spray-drying, emulsification/evaporation, micronisation, etc. The proposals involving lyophilization as part of the procedure which have already been described generally start from mixtures containing organic solvents, water, polymers, excipients, tensoactives, lipids, and lipoproteins among other components. These mixtures in general involve complex emulsification or dilution processes that often consider heating, sonication, intense stirring and the use of polymers specially designed, among other procedures.

Patent applications PCT/ES/2007/070012 and PCT/ES/2007/070013 describe technologies which have simplified the administration process, shortening the preparation time of the formulation for perfusion and decreasing the risks involved, by means of a pharmaceutical formulation of two components: One constituted by a taxane solid composition, especially lyophilized docetaxel and paclitaxel, and the other constituted by a liquid solubilizing composition. Said solid composition of taxane, in particular docetaxel and paclitaxel, has extraordinary solubilization features compared with pharmaceutical actives available on the market. In addition, this solid composition does not contain polyoxyethylated castor oil or polysorbate 80, it can be obtained by a simple procedure since it does not require heating, sonication or intense stirring and it is easy to repeat. Finally, said solid composition is also totally soluble in aqueous solution of only tensoactive in less than a minute.

When dissolving lyophilized taxane in an aqueous dissolving solution of tensoactive, excellent, transparent, stable, low viscosity solutions were obtained, allowing perfect injection into the perfusion bag, in the absence of swelling and presenting optimum chemical and physical characteristics for pharmaceutical use in parenteral infusion. However, foam formation has been detected when mixing said taxane with said solubilizing composition. This foam does not affect the characteristics of the solution but requires a long waiting time for it to disappear before injection. Furthermore, it can hide taxane traces not dissolved, with the resulting therapeutic risks.

The problem of foam in injectable preparations of taxane for infusion has already been informed. For example, the labeling information giving directions for the use of Taxotere™ proposes the mixture of a taxane solution in polysorbate (in the presence of ethanol) with another aqueous solution of dissolving additive and suggests reversing the container without shaking to avoid foam formation, and proposes to let it stand for the foam to disappear. The mixture described is a "dilution" of the active compound (which is in a solution of polysorbate) with water and additive of dilution, to decrease the concentration of docetaxel, and thus avoid the formation of swelling, as it can be seen in patent EP0671912, therefore it does not require stirring. On the contrary, the technical problem solved by the present invention appears when a solid taxane is mixed with a liquid to obtain a stable colloidal solution. This operation is not a dilution, but it constitutes an operation of solubilization or a reconstitution if solid taxane is lyophilized. As it is known, the solubilization of solids generally requires intense stirring, which is not the case in a simple dilution. Hence solubilization of solid taxane in solubilizing compositions comprising tensoactives, such as those described in patent application PCT/ES/2007/070012, requires stirring and causes abundant foam.

This new technical problem related to foam originated when mixing solid taxanes, particularly the lyophilizates such as those described in applications PCT/ES/2007/070012 and PCT/ES/2007/070013 with a solubilizing composition, particularly those described in the applications mentioned, specially solubilizing compositions composed of tensoactive aqueous solution has been neither solved nor stated in the state of the art.

Prior to the appearance of the lyophilized taxanes described, these active matters of poor solubility in water were dissolved in organic solvents such as ethanol, which generated imperceptible or little foam. The possibility of dissolving taxanes directly in tensoactive aqueous solutions in the absence of organic solvent entails the appearance of foam. Bibliography describing various compositions to dissolve taxanes is abundant, as those mentioned in US patent 5.877.205, which uses organic solvent as solubilizing composition. In this document DMA is used as solvent to prepare a liquid formulation of taxanes, especially paclitaxel. DMA is not used as an antifoam agent, and a formulation comprising a solid and a solubilizing composition is neither proposed.

The present invention solves said new technical problem, i.e., it provides solutions in order to avoid or diminish the appearance of foam when a solid composition of taxane is solubilized with an aqueous tensoactive solution.

Another technical problem solved by the present invention is the high overdose of Taxotere formulation: As it can be read in the labeling information giving directions for the use of this product, the amount of active matter is overdosed in 20% due to losses produced by the presence of foam and the final viscosity that renders the availability of the whole active matter impossible. The formulation of the present invention presents a lower viscosity than the corresponding to that of Taxotere, and being free of foam it makes the active matter to be extracted more easily, thus avoiding the need of overdosing.

Besides, the present invention also provides a sterile pre-filled double-chamber syringe. Thus, the preparation of the perfusion formulation is simplified since both chambers come into contact, and by intense stirring the solubilization of the lyophilized solid composition of taxane in said solubilizing composition is achieved, in the absence of foam or substantially free from foam and finally this solution is injected into the perfusion bag or flask containing dextrose or saline solution.

This pre-filled syringe makes the preparation of the perfusion solution a very simple operation, notably reducing the preparation time, eliminating in a large percentage contamination risks both for the product as well as operators and patients, which certainly exist regarding the product available on the market today.

The formulation of the invention would allow eliminating the pre-treatment usually carried out with histamine antagonists, due to the fact that this formulation can be free of polysorbate 80 and polyoxyethylated castor oil. Furthermore, this formulation would allow its administration in a shorter time than usual in the recommended practice, since long periods of perfusion are necessary due to the presence of polysorbate 80. Thus, a shorter time would be needed for administering taxanes using the formulation of the present invention.

Both the decrease of operative preparation time of the perfusion solution as well as the decrease of perfusion time during administration of the formulation of the invention provides the following benefits: a greater number of patients being treated in day care hospitals in the oncological clinical service of the current health system (public and private); reduction of operative costs, on the basis of pharmacoeconomy, due to a better utilization of the health care resources; and giving priority to health care workers involved in said manipulation (the risk of contact of the initial diluting solution with the operators skin and mucosae is almost reduced to 0).

Given that a great number of adverse side effects are attributed to the high concentrations of polysorbate 80, this new formulation could be administered to patients who cannot receive it nowadays, namely patients with renal illnesses, cardiopathies, the elderly, those with polysorbate 80 hypersensitivity,etc.

### Brief Description of the Invention

The pharmaceutical formulation of taxane for the treatment of mammals, preferably humans, object of the present invention comprises two compositions which are mixed before being injected which are:
a. a first solid composition of taxane and
b. a second liquid solubilizing composition comprising a tensoactive and a antifoam agent. That once said compositions a) and b)have been mixed, a liquid essentially free from foam is obtained; where said antifoam agent is chosen from the group comprised by dimethylacetamide, dimethyl sulfoxide, benzyl alcohol, isopropanol, ethanol or a mixture thereof. Preferably, said antifoam agent is found in a concentration of between 2.5% and 60 % in said liquid solubilizing composition; most preferably in a concentration of between 5% and 30 % in said liquid solubilizing composition. Even most preferably, in a concentration of between 10% and 30% in said liquid solubilizing composition.

Preferably, said antifoam agent is dimethylacetamide or a mixture of dimethylacetamide and dimethyl sulfoxide in a concentration of dimethylacetamide in said solubilizing composition of between 5% and 20 % and the concentration of dimethyl sulfoxide in said solubilizing composition of between 0.1 % and 5 %. Another preferred alternative of the invention is ethanol as antifoam agent or a mixture of ethanol and dimethyl sulfoxide.

The tensoactive of the solubilizing composition of the invention is selected from the group comprised by macrogol hydroxystearate, poloxamer, polyvinylpirrolidone, polysorbate, polyethoxylated fatty acids, polyethoxylated vegetable oils or the mixtures thereof; preferably macrogol 15-hydroxystearate; and in a concentration of between 5 % and 60% in said liquid solubilizing composition.

Said liquid solubilizing composition of the invention also comprises up to 90 % of water.

Another preferred formulation of the present invention comprises a liquid solubilizing composition constituted by macrogol 15-hydroxystearate 28%, ethanol as antifoam 28% and water.

In the formulation of the invention, in a preferred embodiment of the present invention, said first solid composition of taxane is lyophilized, preferably from a solution comprising an organic solvent of lyophilization and taxane, preferably free of tensoactives and oils. Said first solid composition of taxane contains less than 5% organic solvent of lyophilization, preferably less than 1%. And said organic solvent of lyophilization is chosen from the group comprised by dioxane, acetic acid, dimethyl sulfoxide or a mixture of thereof. Also preferably, said solution, from which lyophilization is carried out, comprises only said organic solvent and said taxane preferably in the absence of tensoactives, oils, polymers, solubility enhancers, preservatives and excipients.

It is preferred for the formulation of the invention a taxane chosen from the group comprised by derivatives of baccatin III, derivatives of 10-deacethilbaccatine III and conjugates, salts, hydrates and solvates thereof.

On the other hand, said liquid solubilizing composition is, in a preferred embodiment of the present invention, a composition of reconstitution of lyophilizate.

Another object of the present invention is a kit comprising the formulation of the invention to prepare an injectable solution of taxane which comprises: a first container containing said first solid composition of taxane; a second container containing said second liquid solubilizing composition comprising a tensoactive and an antifoam agent; and a syringe; where, preferably, said syringe is pre-filled and comprises said first container and said second container.

Another object of the present invention is a perfusion, or a pharmaceutical solution for perfusion into a patient who needs it, free from foam and precipitates comprising said taxane in a concentration of up to 1.5 mg/ml, tensoactive and antifoam agent in normal saline solution or dextrose solution. Moreover, said perfusion can be obtained by means of mixing said solid composition of taxane a) and said liquid solubilizing composition b) and the subsequent injection of said mixture in normal saline solution or dextrose solution to prepare a transparent parenteral composition for infusion, essentially free from foam, free from swelling and free from precipitates for at least 6 hours. Besides, said perfusion is a colloid of a particle size between 8 and 15 nm.

### Detailed Description of the Invention

On the basis of thorough research and a great number of laboratory tests, a new pharmaceutical formulation of active principles poorly water-soluble, particularly taxanes, which decreases and even avoids foam formation at the moment of preparation when mixing a solid taxane with a solubilizing composition before being injected has been developed. Furthermore, this new formulation allows the preservation of the active matter over long periods of time, maintaining its stability during its useful life, with no need of being kept in cold chain for its preservation, even in tropical and subtropical countries. This new formulation also avoids precipitation and swelling or gelification *in situ,* common problems with some of the formulations of the state of the art occurring upon injection into the perfusion containers containing saline or dextrose solution. In chemotherapy treatments via blood perfusion, this new formulation allows said taxane to be introduced into the body of mammals, especially humans, in the absence of components modifying its solubility or bioavailability, or to form a complex or to bond the drug covalently. The formulation of the invention is also a sterile formulation via a sterilizing filtration process and essentially it does not produce foam at the moment of preparation, prior to infusion. This new pharmaceutical formulation of taxanes, which is the main object of the present invention, comprises on the one hand a solid composition of said taxane, which can be obtained by lyophilization from a solution of organic solvents of lyophilization, and on the other hand, a solubilizing composition of said solid composition comprising a tensoactive and an antifoam agent.

In a preferred embodiment of the present invention, said solid composition of taxane is prepared by lyophilization of a solution of said taxane in a lyophilizing organic solvent. Said lyophilizing organic solvents have a relatively high fusion point, above -10° C to allow solidification and further lyophilization, but below 25° C to allow handling as a liquid at room temperature. Among others, acetic acid, dioxane and dimethylsulphoxide, terbutyl alcohol, etc. can be mentioned as suitable lyophilizing organic solvents which allow easy and rapid solubilization of taxanes, without need of heating, sonicating or intense stirring. Taxanes are soluble in these lyophilizing organic solvents in a wide range of concentrations of up to 50% w/w without precipitation during prolonged periods of time, since a physically and chemically stable solution is produced. Mixtures of these solvents can also be used to realize the present invention.

Taxane solutions in such lyophilizing organic solvents allow, in their manufacturing process, sterilization by filtration through 0.2 µ filter. This filtration is performed prior to lyophilization, obtaining by this operation a sterile powder as the solid composition of the invention. The solubilizing composition is also sterilized by filtration. Thus, sterilization of solids is avoided, which involves complex and expensive procedures, and risks to the drug. Besides, these taxane solutions in lyophilizing organic solvents can be dosed in liquid form in containers to be lyophilized, thus obtaining 5 to 200 mg per container, ready to be reconstituted before injection. The solid lyophilized composition resulting from these taxane solutions in lyophilizing organic solvent is a powder with a large specific surface area which enables a complete and very rapid dissolution in the solubilizing composition. Thus a solid composition is obtained, comprising a lyophilized cake or block, or powder having good stability over a long time at high temperatures, above 25° C, remaining in a proper state without the need of cold chain. Stability tests have been performed, proving that the solid composition of the invention withstands high temperatures, of the order of 60 °C for periods of at least a month with less than 1% degradation.

On the other hand, it has been proved that it is possible to use excipients for lyophilization normally used in the state of the art, such as mannitol, lactose, bile acids, gelatin, etc. The excipient for lyophilization may be added to the taxane solution in lyophilizing organic solvent, either as powder or as an aqueous solution.

Although the addition of water is possible, excipients for lyophilization or an acid (such as citric, lactic, tartaric, ascorbic, acetic, hydrochloric acid or a mixture thereof), it is not essential to achieve the object of the present invention. Consequently, in a preferred embodiment of the present invention, only the taxane solution is lyophilized in lyophilizing organic solvent, without any kind of addition. This allows obtaining a solid composition of taxane with a large specific area, a low apparent density and taxane free from any interaction with other components.

Laboratory tests have proved that the pharmaceutical active substance available on the market, particularly trihydrate or anhydrous docetaxel, cannot be dissolved directly in a tensoactive aqueous solution, and very difficultly in pure polysorbate 80, whereas the solid composition lyophilized is easily soluble both in an aqueous solution of Solutol™ HS 15 as well as in pure polysorbate 80. Also, it has been proved that said solid composition is rapidly soluble in a solution of polysorbate 80 (PS80): EtOH:water (25:9.75:65.25); unlike anhydrous docetaxel (API) available on the market.

An advantage of the solid composition is the remarkable improvement in the readiness to dissolve in solvents of the group of tensoactives and polymers. Thus, said lyophilized solid composition can be easily dissolved in mixtures of Lutrol™ F68, Lutrol™ E400, Solutol™ HS 15, polyethoxylated vegetable oils such as CremophorⓇ and polysorbates, such as Tween 80.

The solid composition obtainable by lyophilization of taxanes in solution of lyophilizing organic solvents, allows the solubilization of said taxanes rapidly both in tensoactive aqueous solutions as well as in pure tensoactives such as polysorbate 80, in the absence of organic solvents added. It also allows the solubilization in aqueous solutions of polymers such as Solutol™ HS15, Lutrol™ F68, povidone, Lutrol™ E400 and mixtures thereof. These formulations can be completely free from ethanol and mainly from polysorbates and polyoxyethylated castor oil.

It must be understood hereby that the solubilization of said lyophilized solid composition of taxane in said solubilizing composition produces a stable colloid, as it has been stated. Said colloid, that can be a colloidal dispersion or a colloidal suspension, presents particle sizes between 8 and 15 nanometers (nm), preferably between 9 and 12 nm. The results obtained can be seen when measuring particle size of said colloids using Zetasizer Nano-ZS (Malvern) equipment, as shown in example 9.

In the state of the art, a usual practice to accelerate the solubilization process of taxanes is the use of organic solvents such as ethanol. This is the most frequently used alternative in the profuse literature related to this technological field. At a first instance, taxane is dissolved in the solvent and at a second stage the amphiphilic polymers, surfactantes or tensoactives and the mixture thereof are added. Generally, long periods of solubilization, sonication, intense stirring, heating and other operations are needed to achieve a stable solution or dispersion.

Solubilization of said solid composition of taxane in a solubilizing composition constituted by water and tensoactive requires stirring and therefore it produces foam. The main object of the present invention is to solve the technical problem of foam. The present invention provides a pharmaceutical formulation of taxane for the treatment of mammals, preferably humans, which comprises two compositions which are mixed before being injected, a first solid composition of taxane and a second liquid solubilizing composition comprising a tensoactive and a antifoam agent.

Besides, the formulation of the invention -once both compositions are mixed- results in a liquid of low viscosity and essentially free from foam which allows the total utilization of the total taxane present, since all the liquid formed is easily extracted from the container.

The tensoactives suitable to be used in the present invention to formulate said solubilizing composition are namely, among others: polyethylene glycol, polyvinylpirrolidone, poloxamers, hydroxypropylcellulose, hydroxyethylcellulose, polymethacrylates, polylysine, polyvynil alcohol, polyacrylic acid, polyethylene oxide, hyaluronic acid, dextran sulphate and its derivatives, calcium stearate, glycerol monostearate, cetostearyl alcohol, emulsifying wax of cetomacrogol, sorbitan esters, alkyl eter of polyethylene oxide, macrogol esters, as cetomacrogol 1000, derivatives of Polyoxyethylene castor oil , polysorbates, polysorbate 80, fatty acid esters of polyoxyethylene sorbitan (TWEEN), polyoxyethylene stearates, sodium dodecyl sulphate, calcium carboxymethylcellulose, sodium carboxymethylcellulose, methylcellulose phthalate of hydroxypropyl methyl cellulose, non crystalline cellulose, Triton.

A preferred embodiment of the present invention comprises as tensoactives for said solubilizing composition Solutol™ HS 15 (Polyethylene glycol 15-hydroxystearate), a macrogol hydroxistearate, Lutrol™ F68, a poloxamer, provided by BASF, polyvinylpirrolidone, or the mixtures thereof. In particular, Solutol™ HS 15, Lutrol™ F68 or the mixtures thereof are preferred.

Solutol™ HS 15 is a Macrogol Hydroxystearate. It is also known by the following names: polyethylene glycol-15-hydroxystearate, polyethylene glycol 660-12-hydroxystearate, macrogol-15-hydroxystearate, CAS No: 70142-34-6 is a polymeric tensoactive used in injectables to solubilize hydrophobic actives substances and avoid sedimentation and recrystallization. Its low toxicity and extraordinary solubilizing power, allow its use in high concentrations. A very low histaminic release has been proved after the administration to mammals as compared to polysorbates ("Applications of Solutol™ HS 15 -A Potent Solubilizer with a Low Toxicity" F. Ruchatz). Some studies suggest that this solubilizing agent can present as desired side effect the reversion of multiple resistance of certain carcinogenic cells regarding anticancer drugs. (K. H. Frömming et al., Acta Pharm. technol. 36 (4), 1990, 214-220; J. S. Coon et al., Cancer Res. 51 (3), 1991, 897-902; J. S. Coon, Proc. Am. Assoc. Cancer Res. 33, 1992, 484; D. Hoover et al., Fundam. Appl. Toxicol. 14 (1990), 589 pp.).

The antifoam agent comprised in the present invention is selected from the group comprised by: Dimethylacetamide (DMA), dimethyl sulfoxide (DMSO), isopropyl alcohol, benzyl alcohol, ethanol and the mixtures thereof, even if other compounds fulfilling this function are able to be used. Surprisingly, both DMA and ethanol have been assayed and it has been determined that they have antifoam effect at very low concentration, from 10%. Particularly, it has been proved that a mixture of DMA and DMSO has a synergic action in reducing foam.

The antifoam agent of the invention is present in a concentration between 5 and 60 % (w/w) in the solubilizing composition, preferably between 7 and 40 %, most preferably between 8 and 30 %, most preferably in a concentration below than 30 %.

In a preferred embodiment of the present invention the antifoam agent is a mixture of DMA 10 % and DMSO 1 % of the solubilizing composition.

In a preferred embodiment of the present invention said solubilizing composition comprises up to 90 % of water, preferably less than 75 %.

In a preferred embodiment of the present invention said solubilizing composition comprises between 5 and 60 % of tensoactive, preferably Solutol, between 5 and 20 % of DMA, optionally between 0.1 and 5 % DMSO, and water.

It has been observed that when the concentration of the antifoam agent increases, solubilization time increases, reaching more than three minutes. This shows that high concentrations of antifoam agent affect the solubilizing capacity of the solubilizing composition of the invention. However, it can be seen in the examples that foam diminishes as the concentration of the antifoam agent increases. A preferred formulation of the present invention contains antifoam agent in a concentration below 30 % of the solubilizing composition.

In a preferred embodiment of the present invention, said formulation is prepared from a solid composition of taxane lyophilized in a sterile container, and a solubilizing composition of Solutol™ HS15 and antifoam agent in water, which after being mixed are injected in a perfusion solution, for example in normal saline solution or dextrose solution, to obtain a taxane solution stable for more than 6 hours, which is infused into patients under oncological treatment.

The present invention also comprises a pharmaceutical perfusion solution containing less than 1 mg/ml of taxane in normal saline solution or dextrose solution, and also contains only Solutol™, and an antifoam agent free from: other tensoactives, oils, other polymers, solubility enhancers, preservatives and excipients. This perfusion solution is the one prepared with the pharmaceutical formulation of taxane of the present invention. Besides, said pharmaceutical perfusion solution for the infusion of taxane in mammals, especially humans, for the treatment of cancer has low toxicity, requires shorter perfusion time (less than 30 minutes) and would not require pre-treatment with steroids or histamine antagonists, since it is free from polysorbate 80, free from polyoxyethylated castor oil, free from emulsions and free from any other component.

The present invention, in another preferred embodiment, comprises a kit consisting of a first container containing the solid composition of lyophilized taxane of the invention; a second container containing the solubilizing composition of the invention; and a syringe.

In another preferred embodiment of the present invention said syringe is pre-filled and contains said containers independent from one another, with means to connect said containers before administration; and optionally, with a filter.

It is worth highlighting that other active principles which are susceptible of being used as active matter in the formulation of the present invention are: albuterol, adapalene, doxazosin mesylate, mometasone furoate, ursodiol, amphotericin, enalapril maleate, felodipine, nefazodone hydrochlorate, valrubicin, albendazole, conjugated estrogens, medroxyprogesterone acetate, nicardipine hydrochlorate, zolpidem tartrate, amlodipine besylate, ethinyl estradiol, omeprazole, rubitecan, amlodipine besylate/benazepril hydrochlorate, etodolac, paroxetine hydrochlorate, atovaquone, felodipine, podofilox, paricalcitol, betamethasone dipropionate, fentanyl, pramipexole dihydrochloride, Vitamin D3 and related analogues, finasteride, quetiapine fumarate, alprostadil candesartan, cilexetil, fluconazole, ritonavir, busulfan, carbamazepine, flumazenil, risperidone, carbidopa/levodopa, ganciclovir, saquinavir, amprenavir, carboplatin, glyburide, sertraline hydrochloride, rofecoxib carvedilol, halobetasolproprionate, sildenafil citrate, celecoxib, chlorthalidone, imiquimod, simvastatin, citalopram, ciprofloxacin, irinotecan hydrochlorate, sparfloxacin, efavirenz, cisapride monohydrate, lansoprazole, tamsulosin hydrochlorate, mofafinil, azithromycin, clarithromycin, letrozole, terbinafine hydrochlorate, rosiglitazone maleate, diclofenac sodium, lomefloxacin hydrochlorate, tirofiban hydrochloride, telmisartan, diazepam, loratadine, toremifene citrate, thalidomide, dinoprostone, mefloquine hydrochloride, trandolapril, docetaxel, mitoxantrone hydrochlorate, tretinoin, etodolac, triamcinolone acetate, estradiol, ursodiol, nelfinavir mesylate, indinavir, beclomethasone dipropionate, oxaprozin, flutamide, famotidine, nifedipine, prednisone, cefuroxime, lorazepam, digoxin, lovastatin, griseofulvin, naproxen, ibuprofen, isotretinoin, tamoxifen citrate, nimodipine, amiodarone, and alprazolam, amphotericin B, cyclosporine, etoposide, topotecan, melphalane, idarubicine, doxorubicin, vinorelbine, vinblastine, vinchristine.

Reference is made herein to concentrations weight/weight (w/w) when no explicit reference is made to any other type of magnitude.

For a better understanding of the technical and functional aspects of the present invention, and without implying a restriction on the scope of this patent application, there follows a set of examples of application involving some of the alternatives comprised in the present invention and a set of comparative examples to assess the differences stated in the light of the prior technique.

### Examples

It is appropriate to highlight that foam above the surface of the solution tested was measured with a caliper. Regarding reports of foam of more than 4 mm it was observed that it occupied the whole surface of the liquid in the container and that it lasted even several hours after the solubilization. However, when 2 mm or less was reported, it was observed that foam only occupied a small edge, which disappeared in a few minutes. Therefore for the aim of this invention, the report of 2 mm of foam responds to a formulation perfectly useful for the purpose of parenteral injection into humans and in accordance with the solution of the technical problem stated in the present invention.

### Example 1

### Obtention of lyophilized docetaxel

Docetaxel (API, raw material) (2.023 g, titre: 98.91%) is dissolved in glacial acetic acid (150 ml) MERCK, lot K37099063 to obtain a solution 13.34 mg/ml. After aseptic filtration, these solutions are dosed in 5 ml vials, 1.5 ml in each vial. Vials are taken to the lyophilizer where they are frozen at -50°C for 240 min, to lyophilize at -5°C for 1500 min, at +5°C for another 1500 min and dry at 30°C, generally more than 1500 min, until residual acetic acid level is lower than 0.5%. Ninety (90) vials containing 20 mg of docetaxel per vial are obtained. Vials are closed with stoppers of bromobuthyl for lyophilization.

### Example 2

Seven (7) vials containing 20 mg of lyophylized docetaxel obtained in example 1 were added with 2.5 ml of the solubilizing composition using a disposable syringe (2.5 ml, Darling, lot 1025) with a disposable hypodermic needle (50x8 - 21Gx2, Neojet, lot 20050925). Said solubilizing composition contains in all cases Solutol 25% and increasing quantities of ethanol as antifoam agent: 0%, 10%, 20%, 30%, 40% and 50 %, and water to complete 100%. Percentages of tensoactive and antifoam agent are always expressed in w/w (weight/weight). The following table shows the results of the test. A remarkable decrease was observed in the foam formed during stirring, necessary for solubilizing the lyophilizate, ranging from 9 mm, measured as the height of foam above the level of liquid, in mm, when only Solutol is used; to almost its disappearance when the solubilizing composition contains between 20% and 30% of antifoam agent. On the other hand, good physical stability is observed, i.e. the solution lasts for more than 6 hs without precipitates. Water used is quality WFI. Ethanol was absolute, Merck, lot K36554486.

**TABLE 1**

| **Solubilizing composition** | **Td** | **Ce** | **Tp** |
|---|---|---|---|
| **Water + 25% Solutol +** | | | |
| 0 % Ethanol | 30" | 9 mm | > 6hs |
| 10 % Ethanol | 1'10" | 4 mm | > 6hs |
| 20 % Ethanol | >3' | 2 mm | > 6hs |
| 25 % Ethanol | 2' | 0 mm | > 6hs |
| 30 % Ethanol | 20" | 0 mm | > 6hs |
| 40 % Ethanol | 15" | 0 mm | > 6hs |
| 50 % Ethanol | 10" | 0 mm | > 6hs |

Complete solubilization of the cake of docetaxel was evidenced in a visual inspection chamber, with black and white background.
Td: Time of solubilization of the lyophilized docetaxel cake in minutes (') or seconds (").
Ce: Quantity of foam. It is worth highlighting that when a height equal to or lower than 2 mm is reported it is because a small quantity of foam is detected only on the edges, reaching the specified height. Said quantity of foam is absolutely compatible with the medical practice and ensures a total availability of the active matter to prepare the subsequent perfusion.
Tp: Precipitation time, i.e. appearance of visible solids of docetaxel.
Solubilization was performed in all cases stirring by inversion at a rate of 1-2 inversions per second until the complete solubilization of the solid inside the vial was observed.

### Example 3

Eight (8) vials containing 20 mg of lyophylized docetaxel obtained in Example 1 were added with 2.5 ml of the solubilizing composition using a disposable syringe (2.5 ml, Darling, lot 1025) with a disposable hypodermic needle (50x8 - 21Gx2, Neojet, lote 20050925). Said liquid solubilizing composition contains water quality WFI, 25% Solutol and increasing quantities of dimethylacetamide(DMA), Merck, lot S4581335 623: 0%, 10%, 20%, 30%, 40% and 50 %. The following table shows the results of the test, where it can be seen that time of solubilization increases with an increase of the concentration of the antifoam agent. A remarkable decrease was observed in the foam formed during stirring, necessary for reconstituting the lyophilizate, ranging from 9 mm, when only Solutol is used, to almost its disappearance when the solubilizing composition contains between 30% and 40% of antifoam agent. On the other hand, it is observed that physical stability, which is maintained for more than 6 hours, is not affected. Solubilization was performed in all cases, stirring by inversion at a rate of 1-2 inversions per second until the complete solubilization of solid inside the vial was observed.

**TABLE 2**

| **Solubilizing composition Water + 25% Solutol +** | **Td** | **Ce** | **Tp** |
|---|---|---|---|
| 0 % DMA | 30" | 9 mm | > 6hs |
| 10 % DMA | 25" | 3 mm | > 6hs |
| 15 % DMA | 30" | 2 mm | > 6hs |
| 20 % DMA | 45 " | 2 mm | > 6hs |
| 15 % DMA | 30" | 2 mm | > 6hs |
| 25 % DMA | >3' | 2 mm | > 6hs |
| 40 % DMA | 20" | 1 mm | > 6hs |
| 50 % DMA | 10" | 0 mm | > 6hs |

The complete solubilization of the docetaxel cake was evidenced in a visual inspection chamber, with black and white background. Td: Time of solubilization of lyophilized docetaxel cake.

Ce: Quantity of foam. It is worth highlighting that when a height equal to or lower than 2 mm is reported it is because a small quantity of foam is detected only on the edges, reaching the specified height. Said quantity of foam is absolutely compatible with the medical practice and ensures a total availability of the active matter to prepare the subsequent perfusion.

Tp: Precipitation time, i.e. appearance of visible solids of docetaxel.

### Example 4

Six (6) vials containing 20 mg of lyophylized docetaxel obtained in Example 1 were added with 2.0 ml of the solubilizing composition using a disposable syringe (2.5 ml, Darling, lot 1025) with a disposable hypodermic needle (50x8 - 21Gx2, Neojet, lot 20050925). Said solubilizing composition contains water quality WFI, Solutol and as antifoam agent:
Dimethyl sulfoxide (DMSO), 0%, 10% and 15 %.
Dimethyl sulfoxide 5% + ethanol 20%
Dimethyl sulfoxide 5% + DMA 10%
Dimethyl sulfoxide 1% + DMA 10%

Percentages of tensoactives and antifoam agents are always expressed in w/w (weight/weight). The following table shows the results of the test.

Solubilization was performed in all cases stirring by inversion at a rate of 1-2 inversions per second until the complete solubilization of solid inside the vial was observed.

**TABLE 3**

| **Solubilizing composition: Water +** | **td** | **Ce** | **tp** |
|---|---|---|---|
| Solutol 30% | 40" | 3 mm | > 8hs |
| Solutol 30% + DMSO 1% | 30" | 3 mm | > 8hs |
| Solutol 30% + DMSO 10% | 2'20" | 3 mm | > 8hs |
| Solutol 30% + DMSO 15% | >3' | 1 mm | > 8hs |
| Solutol 30% +DMSO 5% + Ethanol 20% | 1'10" | 0 mm | > 8hs |
| Solutol 30% + DMSO 5% + DMA 10% | 2'30" | 0 mm | > 8hs |
| Solutol 25% + DMSO 1% + DMA 10% | 30" | 1.5 mm | > 8hs |
| Solutol 25% +DMSO 1% + Ethanol 10% | 2' | 1 mm | > 8hs |

The complete solubilization of the docetaxel cake was evidenced in a visual inspection chamber, with black and white background.
Td: Time of solubilization of lyophilized docetaxel cake.
Ce: Quantity of foam. It is worth highlighting that when a height equal to or lower than 2 mm is reported it is because a small quantity of foam is detected only on the edges, reaching the specified height. Said quantity of foam is absolutely compatible with the medical practice and ensures a total availability of the active matter to prepare the subsequent perfusion.
Tp: Precipitation time, i.e. appearance of visible solids of decetaxel.

### Example 5

Two (2) vials containing 20 mg of docetaxel API (raw material not lyophilized) were added with 2.0 ml of the solubilizing composition using a disposable syringe (2.5 ml, Darling, lot 1025) with a disposable hypodermic needle (50x8-21Gx2, Neojet, lot 20050925). Said liquid solubilizing composition contains water quality WFI: 20 %, Solutol and antifoam agent:
Dimethylacetamide 48 % DMA
Ethanol40 %

Percentages of tensoactives and antifoam agents are always expressed in w/w (weight/weight). Solubilization was performed in all cases stirring by inversion at a rate of 1-2 inversions per second until the complete solubilization of solid inside the vial was observed.

**TABLE 4**

| **Solubilizing composition: Water +** | **td** | **Ce** | **tp** |
|---|---|---|---|
| Solutol 32% + DMA 48 % | 1'45" | 0 mm | > 6hs |
| Solutol 40% + Ethanol 40% | 2'30" | 0 mm | > 6hs |

The complete solubilization of the docetaxel cake was evidenced in a visual inspection chamber, with black and white background.
Td: Time of solubilization of the lyophilized docetaxel cake.
Ce: Quantity of foam. It is worth highlighting that when a height equal or lower than 2 mm is reported it is because a small quantity of foam is detected only on the edges, reaching the specified height. Said quantity of foam is absolutely compatible with the medical practice and ensures a total availability of the active matter to prepare the subsequent perfusion.
Tp: Precipitation time, i.e. appearance of visible solids of decetaxel.

### Example 6

### Chemical stability

The vial of Example 3 containing a solubilizing composition of 15 % DMA was subjected to a chemical stability test described as follows: Immediately after solubilizing docetaxel and at periods of 2, 4, 6 and 8 hours starting from that moment, a sample is taken from the vial using a disposable syringe (1 ml, Darling) with a disposable hypodermic needle (50x8-21Gx2, Neojet). The volume of the sample was 0.5 ml and each sample was weighed and diluted up to a final volume of 10 ml with the mobile phase to be used in HPLC. Reconstituted material remained in a chamber at 25 °C and 60 % room relative humidity during the test time.
Mobile phase: Filtered and deaerated mixture of acetonitrile, Methanol, water (26:32:42 v:v:v).
The column used was Beckman stainless steel (Ultrasphere ODS of 4.6 mm x 25 cm, 5 µm, part N°235329). The guard column used was Beckman (Ultrasphere ODS of 4.6 mm x 4.5 cm, 5 µm, part N° 243533). The detector used was UV type, Waters 2487 dual and the measure was done at wave length 232 nm. The volume injected was 20 µl, using a Waters 717 plus autosampler, and samples were maintained in the autosampler at 4 °C after preparation until the moment of being injected. Retention time for docetaxel is approximately 25 min and run time is 90 min. Flow rate remained 1.0 ml/min using a Waters 1525 binary pump for HPLC. The software used was Breeze version 3.30. The column temperature was 40 °C. A standard for the assessment and determination of related substances was prepared, weighing exactly 20 mg of standard docetaxel in a volumetric flask of 50 ml; it was dissolved with 5 ml ethanol and was diluted up to final volume with mobile phase. A solution of impurity 10-deacethilbaccatine III was prepared in order to detect and quantify it in a chromatogram, weighing 5 mg of 10-deacethilbaccatine III, dissolving with 1 ml ethanol and diluting up to final volume, 10 ml, with mobile phase in a volumetric flask. In the same way, a solution of impurity 7-epi-docetaxel was prepared, weighing 10 mg of docetaxel, 10 ml of NaOH 0.005 N were added. It was left to stand for 2 hours; 2.5 ml of ethanol were added and it was diluted to final volume, 25 ml with mobile phase.

### Results:

**TABLE 5**

| **Time (hours)** | **0** | **2** | **4** | **6** | **8** |
|---|---|---|---|---|---|
| Docetaxel concentration (percentage of area ratio) | 99.61 | 99.59 | 99.57 | 99.57 | 99.54 |

### Example 7

### Reconstitution tests:

Docetaxel solutions obtained in Examples 2 to 5 were chemically and physically stable for periods over 6 hours. In turn, transparent perfusion solutions were obtained with said solutions, useful for oncological treatments, in concentrations near 0.7 mg/ml of docetaxel in normal saline solution or dextrose solution 5%, also physically and chemically stable for more than 6 hours.
These perfusion solutions were obtained taking from the solutions in Examples 2 to 5, a volume such that when injected into 15 ml vials (20 mm opening, type 1 glass, closed with bromobuthyl stoppers and sealed) using a disposable syringe (2.5 ml, Darling, lot 1025) with a disposable hypodermic needle (50x8-21Gx2, Neojet, lot 20050925), a concentration of 0.75 % mg/ml and a final volume of 10 ml was reached. Besides, in all cases, neither foam nor swelling (irreversible gelification) was observed when injecting docetaxel solutions obtained in examples 2 to 5 into physiological solution or saline solution.

### Example 8

One (1) vial containing 20 mg of lyophylized docetaxel of the invention was added with 2.5 ml of the solubilizing composition using a disposable syringe (2.5 ml, Darling, lot 1025) with a disposable hypodermic needle (50x8 - 21Gx2, Neojet, lot 20050925). Said solubilizing composition contains solutol 28 %, ethanol as antifoam agent 28 % and water to complete 100 %. Percentages of tensoactive and antifoam agent are always expressed in w/w (weight/weight). The following table shows the test results.

| **Solubilizing composition** | **Td** | **Ce** | **Tp** |
|---|---|---|---|
| **Water + 28 % Solutol + 28 % Ethanol** | 1 ' | 0 mm | > 6 hours |

It was observed that the solubilizing composition does not improve the solubilization that a solubilizing composition h containing only water and solutol in the absence of antifoam agent has(as the one corresponding to the first test of Example 2, shown in table 1). This is evidenced in the solubilization time, which is 30 seconds when the quantity of ethanol is zero, in the first line of Table 1, whereas the solubilization time in this test, with 28 % of ethanol as antifoam agent in the solubilizing composition takes 1 minute (1').

The complete solubilization of the docetaxel cake was evidenced in a visual inspection chamber, with black and white background.

Td: Time of solubilization of the lyophilized docetaxel cake.

Ce: Quantity of foam. It is worth highlighting that when a height equal or lower than 2 mm is reported it is because a small quantity of foam is detected only on the edges, reaching the specified height. Said quantity of foam is absolutely compatible with the medical, practice and ensures a total availability of the active matter to prepare the subsequent perfusion.

Tp: Precipitation time, i.e. appearance of visible solids of decetaxel.

Solubilization was performed stirring by inversion at a rate of 1-2 inversions per second until the complete solubilization of solid inside the vial was observed.

### Example 9

### ASSESSMENT OF THE PARTICLE SIZE OF THE COLLOID

One (1) vial containing 20 mg of solid composition of lyophylized docetaxel of the invention was added with 2.5 ml of the solubilizing composition using a disposable syringe (2.5 ml, Darling, lot 1025) with a disposable hypodermic needle (50x8 - 21Gx2, Neojet, lot 20050925). Said solubilizing composition contains solutol 28 %, ethanol as antifoam agent 28 % and water to complete 100 %. Percentages of tensoactive and antifoam agent are always expressed in w/w (weight/weight). The solid composition of lyophylized docetaxel is dissolved in said solubilizing composition stirring, and in one minute, a transparent concentrated liquid is obtained in the absence of visible particles and foam. This liquid concentrated is added with the same volume of water. This dilution to 50% in volume is performed in order to measure the particle size of the colloid which is formed.

The Zetasizer Nano-ZS (Malvern) instrument was use for measuring. Work temperature was 20 °C.

Reults:

| **Particle size (nm)** | | |
|---|---|---|
| **Z-ave** | **mean** | **CV %** |
| **9.58** | **9.60** | **0.03** |
| **9.64** | | |
| **9.60** | | |

### Example 10

### ASSESSMENT OF PARTICLE SIZE OF THE COLLOID IN PERFUSION

Two (2) vials containing 20 mg of lyophylized docetaxel of the invention were added each with 2.5 ml of the solubilizing composition using a disposable syringe (2.5 ml, Darling, lot 1025) with a disposable hypodermic needle (50x8 - 21Gx2, Neojet, lot 20050925). Said solubilizing compositions contain solutol 28 %, ethanol as antifoam agent 28 % and water to complete 100 %. Percentages of tensoactive and antifoam agent are always expressed in w/w (weight/weight). The solid compositions of lyophylized docetaxel are dissolved in said solubilizing compositions stirring, and in one minute, a transparent concentrated liquid is obtained in the absence of visible particles and foam. This liquid concentrated is extracted from each vial with a syringe, and one is injected into a bag containing normal saline solution for perfusion and the other is injected into a bag containing dextrose solution for perfusion, reaching the concentrations shown in the following tables. Thus, two perfusion solutions ready to be injected into mammals requiring them are obtained.

Particle size of the colloid formed is measured in a **Zetasizer Nano-ZS (Malvern)** instrument.

### Working temperatures were 20 and 37 °C.

**Perfusion at 0.74 mg/ml in dextrose solution**

| **Particle size (nm)** | | | | | |
|---|---|---|---|---|---|
| **20° C** | | | **37° C** | | |
| **Z-ave** | **mean** | **CV %** | **Z-ave** | **mean** | **CV %** |
| **11.74** | **11.71** | **0.28** | **11.79** | **11.79** | **0.05** |
| **11.72** | | | **11.80** | | |
| **11.67** | | | **11.79** | | |
| **9.23** | | | **10.33** | | |

**Perfusion at 0.74 mg/ml in normal saline solution**

| **Particle size (nm)** | | | **Particle size (nm)** | | |
|---|---|---|---|---|---|
| **20° C** | | | **37° C** | | |
| **Z-ave** | **mean** | **CV %** | **Z-ave** | **mean** | **CV %** |
| **11.51** | **11.50** | **0.10** | **11.79** | **11.76** | **0.24** |
| **11.51** | | | **11.77** | | |
| **11.49** | | | **11.73** | | |

## Claims

1. A pharmaceutical formulation of taxane for the treatment of mammals, preferably humans, with two compositions that are mixed prior to being injected, that comprises:
a. a first solid composition of taxane and
b. a second liquid solubilizing composition comprising a tensoactive and an antifoam agent.

2. The formulation in claim 1 wherein said antifoam agent is selected from the group comprised by dimethylacetamide, dimethylsulphoxide, benzyl alcohol, isopropanol, ethanol or a mixture thereof.

3. The formulation in claim 1 wherein said antifoam agent is dimethylacetamide.

4. The formulation in claim 1 wherein said antifoam agent is a mixture of dimethylacetamide and dimethylsulphoxide.

5. The formulation in claim 4 wherein the concentration of dimethylacetamide in said solubilizing composition is between 5 % and 20 % and the concentration of dimethylsulphoxide in said solubilizing composition is between 0.1 % and 5 %.

6. The formulation in claim 1 wherein said antifoam agent is ethanol.

7. The formulation in claim 1 wherein said antifoam agent is a mixture of ethanol and dimethylsulphoxide.

8. The formulation in claim 1 wherein said antifoam agent is in a concentration of between 2.5% and 60 % in said liquid solubilizing composition.

9. The formulation in claim 1 wherein said antifoam agent is in a concentration of between 5% and 30 % in said liquid solubilizing composition.

10. The formulation in claim 1 wherein said antifoam agent is in a concentration of between 10% and 30% in said liquid solubilizing composition.

11. The formulation in claim 1 wherein said tensoactive is selected from the group comprised by macrogol hydroxystearate, poloxamer, polyvinylpirrolidone, polysorbate, polyethoxylated fatty acids, polyethoxylated vegetable oils or the mixtures thereof.

12. The formulation in claim 1 wherein said tensoactive is in a concentration of between 5 % and 60 % in said liquid solubilizing composition.

13. The formulation in claim 1 wherein said tensoactive is macrogol 15-hydroxystearate.

14. The formulation in claim 1 wherein said liquid solubilizing composition also comprises up to 90 % of water.

15. The formulation in claim 1 wherein said liquid solubilizing composition comprises macrogol 15- hydroxystearate 28 %, ethanol as antifoam agent 28% and water.

16. The formulation in claim 1 wherein once said compositions a) and b) are mixed, a liquid that is essentially free from foam is obtained.

17. The formulation in claim 1 wherein said first solid composition of taxane is liophylized.

18. The formulation in claim 1 wherein said first solid composition of taxane is liophylized from a solution that comprises a lyophilizing organic solvent and a taxane.

19. The formulation in claim 18 wherein said first solid composition of lyophilized taxane contains less than 5% of lyophilizing organic solvent.

20. The formulation in claim 18 wherein said first solid lyophilized composition of taxane contains less than 1% of lyophilizing organic solvent.

21. The formulation in claim 18 wherein said first solid lyophilized composition of taxane is free from tensoactives and oils.

22. The formulation in claim 18 wherein said lyophilizing organic solvent is selected from the group comprised by dioxane, acetic acid, dimethylsulphoxide or a mixture thereof.

23. The formulation in claim 18 wherein said solution comprises only said organic solvent and said taxane in the absence of tensoactives, oils, polymers, solubility enhancers, preservatives and excipients.

24. The formulation according to any of the claims stated above wherein said taxane is selected from the group comprised by derivatives of baccatin III or derivatives from 10-deacetylbaccatin III, conjugates, salts, hydrates and solvates thereof.

25. The formulation according to any of the claims from 1 to 24 wherein said taxane is docetaxel, salts, hydrates or solvates thereof.

26. The formulation according to any of the claims between 1 and 24 wherein said taxane is paclitaxel, salts, hydrates or solvates thereof.

27. The formulation in claim 1 wherein said liquid solubilizing composition is a composition for reconstituting a lyophilizate.

28. A kit of elements comprising the formulation of any of the claims stated above to prepare an injectable solution of taxane **characterized by** comprising: a first container containing said first solid composition of taxane; a second container containing said second liquid solubilizing composition that comprises a tensoactive and an antifoam agent; and a syringe.

29. The kit in claim 28 wherein said syringe is prefilled and comprises said first container and said second container.

30. A taxane perfusion free from foam and precipitates to be injected into a mammal preferably human **characterized by** comprising said taxane in a concentration of up to 1.5 mg/ml, a tensoactive and an antifoam agent in normal saline solution or dextrose solution.

31. A perfusion of taxane free from foam and precipitates to be injected in a mammal preferably human as that in claim 30 **characterized in that** it is obtainable by the mixture of said solid composition of taxane a) and said liquid solubilizing composition b) in claim 1 and the subsequent injection of said mixture in normal saline solution or dextrose solution to form a transparent parental infusion composition, essencially free from foam, free from swelling and free from precipitates for at least 6 hours.

32. A taxane perfusion free from foam and precipitates to be injected into a mammal, preferably human, as in claim 30 **characterized in that** it is a colloid of a particle size ranging from 8 to 15 nm.
